# EUROPÄISCHE PATENTSCHRIFT

(11) **EP 0 730 572 B1**
(45) Veröffentlichungstag und Bekanntmachung des Hinweises auf die Patenterteilung: **24.06.1998**
(21) Anmeldenummer: 95901397.0
(22) Anmeldetag: 19.11.1994
(51) Int. Cl.: C07C 233/36, C11D 1/90, C07C 231/12

(54) **NIEDRIGVISKOSE WÄSSRIGE KONZENTRATE VON BETAINTENSIDEN**
LOW-VISCOSITY AQUEOUS CONCENTRATES OF BETAINE SURFACTANTS
CONCENTRES AQUEUX DE FAIBLE VISCOSITE D'AGENTS TENSIOACTIFS DU TYPE BETAINE

(30) Priorität: 27.11.1993 DE 4340423
(43) Veröffentlichungstag der Anmeldung: 11.09.1996
(73) Patentinhaber: Henkel Kommanditgesellschaft auf Aktien, 40191 Düsseldorf (DE)
(72) Erfinder: UPHUES, Günter, D-40789 Monheim (DE); NEUMANN, Peter, D-40589 Düsseldorf (DE); BEHLER, Ansgar, D-46230 Bottrop (DE)
(86) Internationale Anmeldenummer: EP9403830
(87) Internationale Veröffentlichungsnummer: WO9514658

(56) Entgegenhaltungen:
- EP-A- 0 353 580
- DE-C- 4 207 386
- US-A- 4 246 131

## Beschreibung

### Gebiet der Erfindung

Die Erfindung betrifft niedrigviskose wäßrige Konzentrate von Betaintensiden mit einem Gehalt an ausgesuchten Polyolverbindungen sowie ein Verfahren zu ihrer Herstellung.

### Stand der Technik

Betaine bzw. amphotere Tenside sind ausgesprochen hautverträglich und weisen ausgezeichnete Reinigungseigenschaften auf. Sie eignen sich daher in besonderer Weise zur Konfektionierung einer Vielzahl von oberflächenaktiven Produkten. Zu ihrer Herstellung geht man im einfachsten Fall von tertiären Aminen aus, die mit Natriumchloracetat zu Alkylbetainen umgesetzt werden. Die Umsetzung von Fettsäureaminoamiden oder Imidazolinen mit Natriumchloracetat führt zur Bildung von amphoteren Tensiden vom Typ der Glycinate; wird als Alkylierungsmittel Acrylsäureester eingesetzt, bilden sich Aminopropionate. Verbindungen der genannten Art sind in einer Vielzahl von Übersichtsartikeln beschrieben, von denen an dieser Stelle nur **Parf.Cosm.Arm. 70, 67 (1986), HAPPI, 70**, **(Nov.1986)** und **Soap Coam.Chem.Spec. 46, (Apr.1990)** genannt sein sollen.

Ein besonderes Anliegen bei der Herstellung der Betaine bzw. amphoteren Tenside besteht darin, möglichst reine und somit dermatologisch und toxikologisch unbedenkliche Produkte zur Verfügung zu stellen. Unerwünscht sind beispielsweise Spuren von freien Aminen, Chloressigsäure und insbesondere Dichloressigsäure in den Tensiden. Auch Konservierungsstoffe, die die Betaine bzw. amphoteren Tenside vor mikrobiellem Befall schützen sollen, sind häufig nicht erwünscht, so daß ein weiteres Bedürfnis nach Produkten besteht, die auch ohne Zusatz von Hilfsstoffen gegenüber Keimbefall stabilisiert sind. Eine dritte Aufgabe der Erfindung besteht schließlich darin, möglichst hellfarbige Produkte mit einem hohen Feststoffgehalt, vorzugsweise im Bereich von 40 bis 50 Gew.-% zur Verfügung zu stellen.

Aus dem Stand der Technik sind bereits eine Reihe von Druckschriften bekannt, die Teillösungen für die kumulierte Aufgabenstellung anbieten.

So wird beispielsweise in der **DE-A1 39 39 264** (Henkel) vorgeschlagen, den Gehalt an Chloressigsäure in amphoteren Tensiden durch eine nachträgliche Behandlung der wäßrigen Lösungen mit Ammoniak, Aminosäuren oder Oligopeptiden zu verringern. Aus der **DE-OS 29 26 479** (Th.Goldschmidt) ist ein Verfahren bekannt, bei dem man die Quaternierung im pH-Bereich von 7,5 bis 10,5 durchführt und so den Restgehalt an freiem Alkylierungsmittel minimiert. In die gleiche Richtung weist die Lehre der **DE-A 20 63 424** (Rewo), die die pH-Regulierung für die Alkylierung von Imidazolinen beschreibt. Ferner wird in der **DE-C 37 26 322** (Th.Goldschmidt) ein Verfahren zur Nachbehandlung von Betainen beschrieben, bei dem man den Stoffen Mineralsäuren in solchen Mengen zusetzt, daß der pH-Wert der Lösung 1 bis 4,5 beträgt. Auf den Gehalt an Dichloressigsäure haben diese Verfahren jedoch keinen Einfluß. In der **DE-A1 42 05 880** (Th.Goldschmidt) wird zur Minimierung von chlorierten Verunreigungen vorgeschlagen, die Betaine in wäßriger Lösung bei einer Temperatur im Bereich von 115 bis 180°C und damit unter erhöhtem Druck durchzuführen. Schließlich sind aus der **DE-C1 42 07 386** (Th.Goldschmidt) Betainkonzentrate mit Feststoffgehalten oberhalb von 40 Gew.-% bekannt, die 1 bis 3 Gew.-% freie Fettsäure und 0 bis 4 Gew.-% Glycerin enthalten, einen Gehalt an freiem Amidoamin von weniger als 1 Gew.-% und einen pH-Wert im Bereich von 5 bis 8 aufweisen.

Nachdem keines dieser Verfahren des Stands der Technik die kumulierte Aufgabenstellung zufriedenstellend zu lösen vermag, hat die Aufgabe der Erfindung darin bestanden, neue wäßrige Betaine zur Verfügung zu stellen, die fließ- und pumpfähig sind, auch bei längerer Lagerung nicht vergelen, einen Feststoffgehalt von mindestens 40 Gew.-% und einen minimierten Gehalt an unerwünschten Nebenbestandteilen, insbesondere chlorierten Stoffen und freien Aminen aufweisen.

### Beschreibung der Erfindung

Gegenstand der Erfindung sind niedrigviskose wäßrige Konzentrate von Betaintensiden der Formel (I), 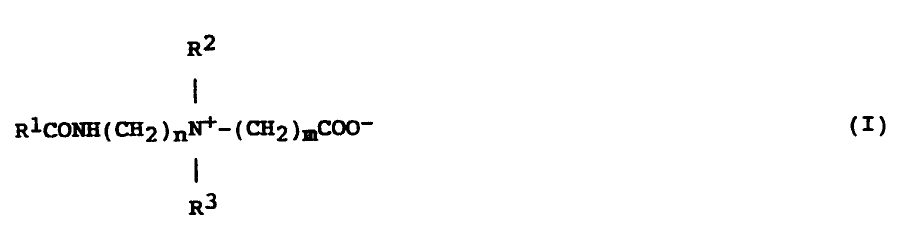 in der R¹CO für einen aliphatischen Acylrest mit 8 bis 22 Kohlenstoffatomen, R² und R³ unabhängig voneinander für einen Alkylrest mit 1 bis 4 Kohlenstoffatomen und n und m unabhängig voneinander für Zahlen im Bereich von 1 bis 5 steht, erhältlich durch Kondensation von Fettsäureaminoamiden mit Halogencarbonsäuresalzen in Gegenwart von Verflüssigungsmitteln, die sich dadurch auszeichnen, daß sie einen Gehalt an Polyolverbindungen ausgewählt aus der Gruppe, die gebildet wird von Alkylenglycolen, technischen Oligoglyceringemischen, Methyolverbindungen, Niedrigalkylglucosiden, Zuckeralkoholen, Zuckern und Aminozuckern, aufweisen.

Überraschenderweise wurde gefunden, daß der Zusatz der genannten Polyolverbindungen die Viskosität der Konzentrate so weit herabsetzt, daß nunmehr fließ- und pumpfähige Produkte mit einem Feststoffgehalt im Bereich von 40 bis 55 Gew.-% zur Verfügung gestellt werden können, die auch bei längerer Lagerung keine Tendenz zur Vergelung zeigen. Die erfindungsgemäßen Konzentrate sind zudem hell farbig und weisen einen minimierten Gehalt an chlorierten Verbindungen auf. Zur Sicherstellung einer ausreichend niedrigen Viskosität werden weder bestimmte Gehalte an freier Fettsäure noch an freiem Aminoamid benötigt. Zudem sind die Konzentrate auch ohne Zusatz von Konservierungsmitteln gegen antimikrobiellen Befall ausreichend geschützt.

### Betaine

Betaine, genauer Fettsäureamidobetaine, stellen bekannte Stoffe dar. Vorzugsweise bezieht sich die Erfindung auf wäßrige Konzentrate von Betaintensiden, die der Formel (I) folgen, in der R¹CO für einen Acylrest mit 8 bis 18 Kohlenstoffatomen, R¹ und R² für jeweils eine Methylgruppe und n und m unabhängig voneinander für 2 oder 3 steht.

### Polyolverbindungen

Als verflüssigende Polyolverbindungen kommen in Betracht:
a) Alkylenglycole wie beispielsweise Ethylenglycol, Diethylenglycol, Propylenglycol;
b) technische Oligoglyceringemische mit einem Eigenkondensationsgrad von 1,5 bis 10 wie etwa technische Diglyceringemische mit einem Diglyceringehalt von 40 bis 50 Gew.-%;
c) Methyolverbindungen, wie insbesondere Trimethylolethan, Trimethylolpropan, Trimethylolbutan, Pentaerythrit und Dipentaerythrit;
d) Niedrigalkylglucoside, insbesondere solche, mit 1 bis 8 Kohlenstoffen im Alkylrest wie beispielsweise Methyl- und Butylglucosid;
e) Zuckeralkohole mit 5 bis 12 Kohlenstoffatomen wie beispielsweise Sorbit oder Mannit,
f) Zucker mit 5 bis 12 Kohlenstoffatomen wie beispielsweise Glucose oder Saccharose;
g) Aminozucker wie beispielweise Glucamin.

Üblicherweise werden die Polyolverbindungen in Mengen von 1 bis 10, vorzugsweise 1,5 bis 3,0 Gew.-% - bezogen auf die Konzentrate - eingesetzt. Obschon die verflüssigenden Polyolverbindungen den Konzentraten auch nachträglich zugesetzt werden können, hat es sich als vorteilhaft erwiesen, die Stoffe schon während der Kondensationsreaktion zuzusetzen.

### Betainkonzentrate

Die erfindungsgemäßen Betainkonzentrate weisen einen Feststoffgehalt von mindestens 40, vorzugsweise von 40 bis 55 und insbesondere von 42 bis 48 Gew.-% - bezogen auf die Konzentrate - auf. Der Aktivsubstanzgehalt, d.h. der Gehalt an Betainen, liegt in der Regel 8 bis 12 Gew.-% niedriger.

Im Hinblick auf die Lagerstabilität der Produkte hat es sich als vorteilhaft erweisen, die Konzentrate auf einen pH-Wert im Bereich von 5 bis 8 einzustellen.

Der Gehalt an freiem Fettsäureaminoamid liegt üblicherweise unter 1 und insbesondere unter 0,5 Gew.-%, der Gehalt an Mono- bzw. Dichloressigsäure jeweils unter 5 ppm - bezogen auf die Konzentrate. Vorzugsweise sind die Konzentrate frei von Glycerin.

### Kondensationsreaktion

Ein weiterer Gegenstand der Erfindung betrifft ein Verfahren zur Herstellung niedrigviskoser wäßriger Konzentrate von Betaintensiden, bei dem man Fettsäureaminoamide der Formel (II), 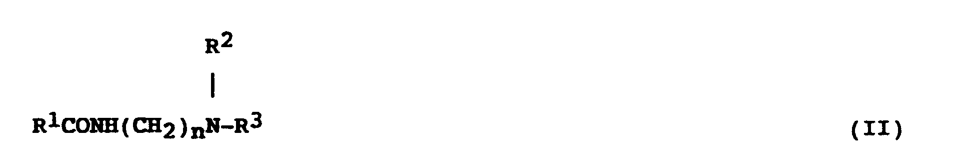 in der R¹CO für einen aliphatischen Acylrest mit 8 bis 22 Kohlenstoffatomen, R² und R³ unabhängig voneinander für einen Alkylrest mit 1 bis 4 Kohlenstoffatomen und n für Zahlen im Bereich von 1 bis 5 steht, mit Halogencarbonsäuren bzw. deren Salzen der Formel (III) kondensiert,

**X-(CH**_{**2**}**)**_{**m**}**COOY** (III)

in der X für ein Halogen, Y für Wasserstoff oder ein Alkalimetall und m für Zahlen im Bereich von 1 bis 5 steht, wobei man die Reaktion in Gegenwart von Polyolverbindungen ausgewählt aus der Gruppe, die gebildet wird von Alkylenglycolen, technischen Oligoglyceringemischen, Methyolverbindungen, Niedrigalkylglucosiden, Zuckeralkoholen, Zuckern und Aminozukkern, als Verflüssigungsmitteln durchführt.

Die Durchführung der Kondensationsreaktion erfolgt in an sich bekannter Weise, wobei man das Fettsäureaminoamid, vorzugsweise C₁₂/₁₈- bzw. C₈/₁₈-Kokosfettsäureaminoamide, und die Halogencarbonsäuren bzw. deren Salze, vorzugsweise Natriumchloracetat, unter Einhaltung eines pH-Wertes im Bereich von 7 bis 8 erhitzt und nachdem der Gehalt an freiem Aminoamid unter 0,5 Gew.-% abgesunken ist, die Reaktionsmischung in einem Druckgefäß über einen Zeitraum von 1 bis 2 h einer Nachbehandlung bei einer Temperatur von 100 bis 130°C und einem pH = 10 bis 14 unterwirft. Nach Abschluß der Nachreaktion empfiehlt es sich, das Konzentrat wieder auf einen neutralen pH-Wert einzustellen.

### Gewerbliche Anwendbarkeit

Die erfindungsgemäßen wäßrigen Betaine sind auch hochkonzentriert flüssig, lagerstabil und weisen einen minimierten Anteil an unerwünschten Nebenbestandteilen auf. Sie eignen sich für die Herstellung oberflächenaktiver Mittel, insbesondere von Reinigungsprodukten sowie Haarbehandlungs- und -pflegemitteln, in denen sie in Mengen von 1 bis 30, vorzugsweise 2 bis 10 Gew.-% - bezogen auf die Mittel - enthalten sein können.

Die folgenden Beispiele sollen den Gegenstand der Erfindung näher erläutern, ohne ihn darauf einzuschränken.

### Beispiele

**Allgemeine Herstellvorschrift**. 130,7 g (1,1 mol) Natriumchloracetat wurden zusammen mit 1,8 bis 2,5 Gew.-% - bezogen auf die resultierenden Konzentrate - eines Polyols in ca. 500 ml Wasser gelöst. Anschließend wurden bei 40°C 213 g (0,7 mol) eines Fettsäureaminoamids eingerührt, das durch Amidierung einer gehärteten Kokosfettsäure mit 3-N,N-Dimethylaminopropylamin hergestellt worden war und einen Gehalt von 4,6 Gew.-% titrierbaren Stickstoff aufwies.

Nach Erwärmen des Gemisches auf 90°C und Bildung einer klaren, dünnflüssigen Lösung wurden weitere 91,3 g (0,3 mol) des Fettsäureaminoamids zugegeben. Unter Einhaltung eines pH-Wertes zwischen 7,5 und 8,0 (Verbrauch an 37 Gew.-%iger Natronlauge : 10,7 g) wurde die Reaktion fortgesetzt, bis nach ca. 2 h der Gehalt an freien Aminfunktionen unter 0,5 mmol/ 100 g, d.h. < 0,15 % Fettsäureaminoamid (ermittelt durch HPLC-Analyse) abgesunken war.

Dem Ansatz wurden nun entsprechend einem pH-Wert von 12,5 in 10 Gew.-%iger Produktlösung 43,2 g 37 Gew.-%ige Natronlauge zugesetzt und das Produkt in einer Druckapparatur 1 h bei 120°C gerührt. Nach der Abkühlung wurde der Produkt-pH-Wert mit 53,8 g 24-Gew.-%iger Salzsäure auf 7,0 eingestellt. Die bei Raumtemperatur klaren, dünnflüssigen Lösungen wiesen die Kenndaten gemäß Tab.1 auf:

**Tab. 1:**

| Versuchsergebnisse Prozentangaben als Gew.-% | | | | | | | | |
|---|---|---|---|---|---|---|---|---|
| Bsp. | P | P % | MCE ppm | DCE ppm | GS % | S % | FFA % | FSG % |
| 1 | A | 1,8 | 1,8 | 2,3 | 0,8 | 7,8 | 0,1 | 44,1 |
| 2 | A | 2,5 | 1,8 | 2,4 | 0,7 | 8,5 | 0,1 | 48,2 |
| 3 | B | 2,0 | 1,9 | 2,2 | 0,8 | 7,9 | 0,1 | 44,0 |
| 4 | C | 2,0 | 1,8 | 2,3 | 0,8 | 8,1 | 0,1 | 43,8 |
| 5 | D | 2,0 | 1,8 | 2,4 | 0,8 | 7,8 | 0,1 | 44,1 |
| 6 | E | 2,0 | 1,9 | 2,5 | 0,9 | 7,7 | 0,1 | 43,9 |
| 7 | F | 2,0 | 1,8 | 2,3 | 0,8 | 8,0 | 0,1 | 43,5 |
| V1 | G | 2,0 | 1,9 | 2,2 | 0,9 | 7,8 | 0,1 | 42,0 |

Legende: P = Polyol
A) Sorbit
B) Glucose
C) Trimethylolpropan
D) Pentaerythrit
E) Butylglucosid
F) Glucamin
G) Glycerin

- MCE =: Monochloressigsäure
- DCE =: Dichloressigsäure
- GS =: Glycolsäure
- FFA =: Freies Aminoamid
- S =: Salz (=NaCl)
- FSG =: Feststoffgehalt

### Diskussion der Beispiele

Die Betainkonzentrate der erfindungsgemäßen Beispiele 1 bis 7 wiesen einen Feststoffgehalt oberhalb von 40 Gew.-% auf und lagen als niedrigviskose, leicht gießbare und pumpfähige klare Flüssigkeiten mit ausgezeichneter Lagerstabilität vor. Bei Verwendung von Glycerin als Verflüssiger (Vergleichsbeispiel V1) wurde zwar ebenfalls ein Produkt mit einem Feststoffgehalt oberhalb von 40 Gew.-% erhalten, das jedoch bei der Drucknachbehandlung vergelte und danach weder fließ- noch pumpfähig war.

Ohne Zusatz der erfindungsgemäß einzusetzenden Polyole vergelten alle Produkte schon zu Beginn der Kondensationsreaktion, spätestens jedoch während der Drucknachbehandlung. Es konnten nur Betaine mit einem Feststoffgehalt von ca. 35 Gew.-% erhalten werden.

## Patentansprüche

1. Niedrigviskose wäßrige Konzentrate von Betaintensiden der Formel (I) 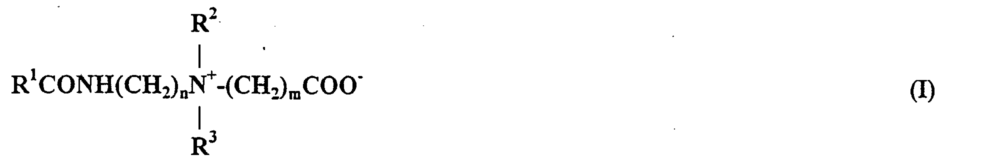 in der R¹CO für einen aliphatischen Acylrest mit 8 bis 22 Kohlenstoffatomen, R² und R³ unabhängig voneinander für einen Alkylrest mit 1 bis 4 Kohlenstoffatomen und n und m unabhängig voneinander für Zahlen im Bereich von 1 bis 5 stehen, erhältlich durch Kondensation von Fettsäureaminoamiden mit Halogencarbonsäuresalzen in Gegenwart von Verflüssigungsrnitteln sowie anschließender Drucknachbehandlung bei einer Temperatur von 110 bis 130°C und einem pH-Wert von 10 bis 14, **dadurch gekennzeichnet,** daß sie einen Gehalt an Polyolverbindungen aufweisen, die ausgewählt sind aus der Gruppe, die von Oligoglyceringemischen, Methylolverbindungen, Niedrigalkylglucosiden, Zuckeralkoholen, Zuckern und Aminozuckern gebildet wird.

2. Konzentrate nach Anspruch 1, **dadurch gekennzeichnet,** daß die Betaintenside der Formel (I) folgen, in der R¹CO für einen Acylrest mit 8 bis 18 Kohlenstoffatomen; R¹ und R² für jeweils eine Methylgruppe und n und m unabhängig voneinander für 2 oder 3 steht.

3. Konzentrate nach den Ansprüchen 1 und 2, **dadurch gekennzeichnet,** daß der Gehalt an Polyolverbindungen im Bereich von 1 bis 10 Gew.-% - bezogen auf die Konzentrate - liegt.

4. Konzentrate nach den Ansprüchen 1 bis 3, **dadurch gekennzeichnet**, daß der Feststoffanteil im Bereich von 40 bis 55 Gew.-% - bezogen auf die Konzentrate - liegt.

5. Konzentrate nach den Ansprüchen 1 bis 4, **dadurch gekennzeichnet,** daß der pH-Wert während der Kondensationsreaktion im Bereich von 5 bis 8 liegt.

6. Konzentrate nach den Ansprüchen 1 bis 5, **dadurch gekennzeichnet,** daß der Gehalt an freiem Aminoamid unter 1 Gew.-% - bezogen auf die Konzentrate - liegt.

7. Konzentrate nach den Ansprüchen 1 bis 6, **dadurch gekennzeichnet,** daß der Gehalt an Mono- bzw. Dichloressigsäure jeweils unter 5 ppm - bezogen auf die Konzentrate - liegt.

8. Konzentrate nach den Ansprüchen 1 bis 7, **dadurch gekennzeichnet,** daß die Produkte frei von Glycerin sind.

9. Verfahren zur Herstellung niedrigviskoser wäßriger Konzentrate von Betaintensiden, bei dem man Fettsäureaminoamide der Formel (**II**), 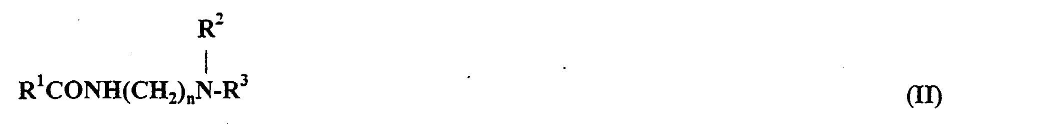 in der R¹CO für einen aliphatischen Acylrest mit 8 bis 22 Kohlenstoffatomen, R² und R³ unabhängig voneinander für einen Alkylrest mit 1 bis 4 Kohlenstoffatomen und n für Zahlen im Bereich von 1 bis 5 steht, mit Halogencarbonsäuren bzw. deren Salzen der Formel (**III**) kondensiert,
**X-(CH**_{**2**}**)**_{**m**}**COOY (III)**
in der X für ein Halogen, Y für Wasserstoff oder ein Alkalimetall und m für Zahlen im Bereich von 1 bis 5 steht und anschließend einer Drucknachbehandlung bei Temperaturen im Bereich von 100 bis 130°C und einem pH-Wert von 10 bis 14 unterwirft, **dadurch gekennzeichnet**, daß man die Kondensationsreaktion in Gegenwart von Polyolverbindungen durchführt, die ausgewählt sind aus der Gruppe, die gebildet wird von Oligoglyceringemischen. Methylolverbindungen, Niedrigalkylglucosiden, Zuckeralkoholen, Zuckern und Aminozuckern.

## Claims

1. Low viscosity water-containing concentrates of betaine surfactants corresponding to formula (**I**): 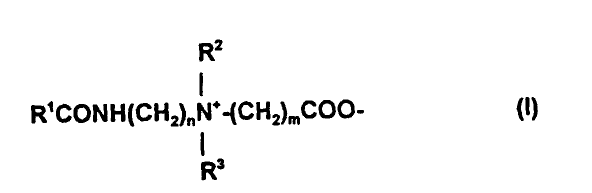 in which R¹CO is an aliphatic acyl radical containing 8 to 22 carbon atoms, R² and R³ independently of one another represent an alkyl radical containing 1 to 4 carbon atoms and n and m independently of one another are numbers of 1 to 5,
obtainable by condensation of fatty acid aminoamides with halocarboxylic acid salts in the presence of liquefying agents and subsequent pressure treatment at a temperature of 110 to 130°C and a pH value of 10 to 14, characterized in that they contain polyol compounds selected from the group consisting of oligoglycerol mixtures, methylol compounds, lower alkyl glucosides, sugar alcohols, sugars and amino sugars.

2. Concentrates as claimed in claim 1, characterized in that the betaine surfactant correspond to formula (**I**), in which R¹CO is an acyl radical containing 8 to 18 carbon atoms, R¹ and R² each represent a methyl group and n and m independently of one another have a value of 2 or 3.

3. Concentrates as claimed in claims 1 and 2, characterized in that the content of polyol compounds is in the range from 1 to 10% by weight, based on the concentrates.

4. Concentrates as claimed in claims 1 to 3, characterized in that the solids content is in the range from 40 to 55% by weight, based on the concentrates.

5. Concentrates as claimed in claims 1 to 4, characterized in that the pH value during the condensation reaction is in the range from 5 to 8.

6. Concentrates as claimed in claims 1 to 5, characterized in that the content of free aminoamide is less than 1% by weight, based on the concentrates.

7. Concentrates as claimed in claims 1 to 6, characterized in that the content of monochloroacetic acid or dichloroacetic acid is below 5 ppm, based on the concentrates.

8. Concentrates as claimed in claims 1 to 7, characterized in that the products are free from glycerol.

9. A process as for the production of low-viscosity water-containing concentrates of betaine surfactants, in which fatty acid amino amides corresponding to formula (**II**): 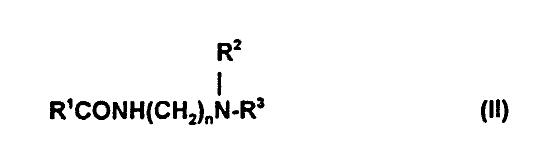 in which R¹CO is an aliphatic acyl radical containing 8 to 22 carbon atoms, R² and R³ independently of one another represent an alkyl radical containing 1 to 4 carbon atoms and n is a number of 1 to 5,
are condensed with halocarboxylic acids or salts thereof corresponding to formula (**III**):
**X-(CH**_{**2**}**)**_{**m**}**COOY (III)**
in which X is a halogen atom, Y is hydrogen or an alkali metal and m assumes a value of 1 to 5,
and then subjected to a pressure treatment at temperatures of 100 to 130°C and a pH value of 10 to 14, characterized in that the condensation reaction is carried out in the presence of polyol compounds selected from the group consisting of oligoglycerol mixtures, methylol compounds, lower alkyl glucosides, sugar alcohols, sugars and amino sugars.

## Revendications

1. Concentrés aqueux faiblement visqueux d'agents actifs du type bétaine de formule (I) : 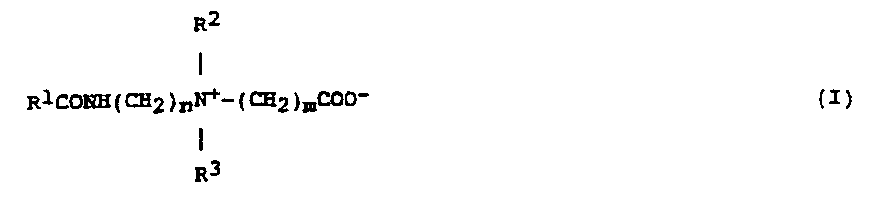 dans laquelle R¹CO représente un radical acyle aliphatique ayant de 8 à 22 atomes de carbone, R² et R³, indépendamment l'un de l'autre représentent un radical alkyle ayant de 1 à 4 atomes de carbone et n et m indépendamment l'un de l'autre représentent des nombres dans la zone de 1 à 5, que l'on peut obtenir par condensation d'aminoamides d'acide gras avec des sels d'acide halogénocarboxylique en présence d'agents de fluidification ainsi que par retraitement sous pression consécutif, à une température de 110 à 130°C et à une valeur de pH de 10 à 14,
caractérisés en ce qu'
ils possèdent une teneur en composés polyol qui sont choisis dans le groupe formé des mélanges d'oligoglycérols, des composés du type méthylol, des (alkyl inférieur)glucosides, des alcools de sucre, des sucres et des aminosucres.

2. Concentrés selon la revendication 1,
caractérisés en ce que
les agents tensioactifs du type bétaine répondent à la formule (I), dans laquelle R¹CO représente un reste acyle ayant de 8 à 18 atomes de carbone, R¹ et R² respectivement un groupe méthyle, et n et m indépendamment l'un de l'autre représentent 2 ou 3.

3. Concentrés selon les revendications 1 et 2,
caractérisés en ce que
la teneur en composés polyol se situe dans la zone de 1 à 10 % en poids, rapporté aux concentrés.

4. Concentrés selon les revendications 1 à 3,
caractérisés en ce que
la quantité de matière solide se situe dans la zone de 40 à 55 % en poids, rapporté aux concentrés.

5. Concentrés selon les revendication 1 à 4,
caractérisés en ce que
la valeur du pH pendant la réaction de condensation se situe dans la plage de 5 à 8.

6. Concentrés selon les revendications 1 à 5,
caractérisés en ce que
la teneur en aminoamide libre se situe en dessous de 1 % en poids, rapporté aux concentrés.

7. Concentrés selon les revendications 1 à 6,
caractérisés en ce que
la teneur en acide mono- ou dichloroacétique se situe respectivement en dessous de 5 ppm, rapportés aux concentrés.

8. Concentrés selon les revendications 1 à 7,
caractérisés en ce que
les produits sont exempts de glycérol.

9. Procédé d'obtention de concentrés aqueux faiblement visqueux d'agents tensioactifs du type bétaïne, dans lequel on condense des aminoamides d'acide gras de formule (II) : 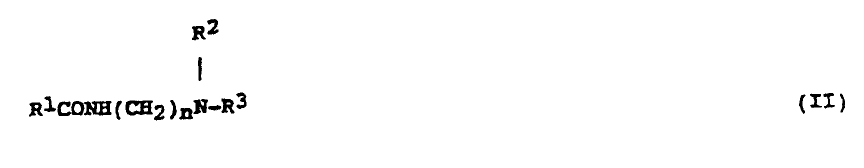 dans laquelle R¹CO représente un radical acyle aliphatique ayant de 8 à 22 atomes de carbone, R² et R³ indépendamment l'un de l'autre représentent un radical alkyle ayant de 1 à 4 atomes de carbone, et n représente des nombres dans la zone de 1 à 5, avec des acides halogéno carboxyliques ou leurs sels de formule (III) :
**X-(CH**_{**2**}**)mCOOY (III)**
dans laquelle X représente un halogène, Y représente de l'hydrogène ou un métal alcalin et m représente des nombres dans la zone de 1 à 5,
et ensuite soumet à un retraitement sous pression à des températures dans la zone de 100 à 130°C, et à une valeur de pH de 10 à 14,
caractérisé en ce qu'
on effectue la réaction de condensation en présence de composés polyol qui sont choisis dans le groupe formé des mélanges d'oligoglycérols, des composés de méthylol, des (alkyl inférieur)glucosides, des alcools de sucre, des sucres et des aminosucres.
